(19) Europäisches Patentamt
European Patent Office
Office européen des brevets

(11) Publication number: **0 081 756**
**B1**

(12) # EUROPEAN PATENT SPECIFICATION

(45) Date of publication of patent specification: **15.05.85**

(51) Int. Cl.[4]: **C 07 D 213/75,** A 61 K 31/44

(21) Application number: **82111143.2**

(22) Date of filing: **02.12.82**

(54) **New compounds with antiinflammatory and antitussive activity, process for their preparation and relative pharmaceutical compositions.**

(30) Priority: **14.12.81 IT 2557081**
**08.11.82 IT 2411682**

(43) Date of publication of application:
**22.06.83 Bulletin 83/25**

(45) Publication of the grant of the patent:
**15.05.85 Bulletin 85/20**

(84) Designated Contracting States:
**AT BE CH DE FR GB IT LI LU NL SE**

(56) References cited:
**FR-A-1 584 802**

**E. SCHRÖDER et al., Arzneimittelchemie II, Georg Thieme Verlag, Stuttgart, 1976, Seite 319, Verbindung 13**

(73) Proprietor: **MEDEA RESEARCHES S.r.l.**
**Via Cappuccini, 20**
**I-20100 Milan (IT)**

(72) Inventor: **Quadro, Giuseppe**
**Via Pisacane, 34/A**
**I-20100 Milan (IT)**

(74) Representative: **Bianchetti, Giuseppe**
**Studio Consulenza Brevettuale Via Senato 24**
**I-20121 Milan (IT)**

**EP 0 081 756 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a series of N-pyridyl-amides of 1-phenyl-cyclopentancarboxylic acid having formula (I)

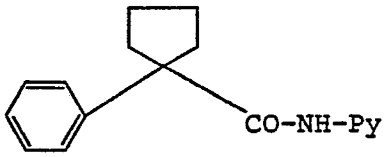

(I)

wherein Py is the pyridine ring, optionally substituted with one or more methyl groups or chlorine atoms.

Compounds (I) proved to be endowed with interesting pharmacological properties, which make them suitable for the therapeutic use as antiinflammatory agents.

The invention relates therefore also to pharmaceutical compositions with antiinflammatory activity containing as active principle one or more amides of formula (I).

Object of the invention is finally provided by a process for the preparation of N-pyridylamides (I), characterized in that activated derivatives of 1-phenyl-cyclopentancarboxylic acid, having general formula II, are reacted with aminopyridines III, according to the scheme:

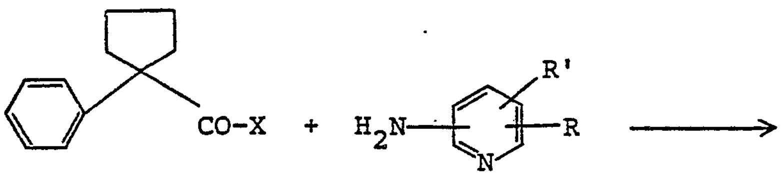

(I)

(II) (III)

In such a scheme, X is an halogen atom, or an alcoxy group, or a residue transforming (II) in a symmetrical or mixed anhydride; R and R', which can be the same or different, are hydrogen atoms, methyl groups or chlorine atoms.

The reaction is preferably carried out with a nearly equimolar ratio between (II) and (III); it is possible to operate in the absence of solvents, or preferably in solvents inert in the reaction conditions (such as ethyl ether, dioxane, chloroform, benzene or toluene), at temperatures ranging from 0°C and the solvent's boiling point. Whereas X is halogen, it is convenient to use an excess of aminopyridine III as a hydrohalic acid acceptor.

The same purpose can be achieved by operating in presence of alkaline or alkaline-earth metals carbonate or bicarbonate, or in presence of tertiary bases.

The following examples illustrate the process according to the invention.

### Example 1

3-[(1-Phenyl)-cyclopentyl-carbonyl]aminopyridine

60 Mmoles of 3-aminopyridine, 50 ml of anhydrous benzene and 50 mmoles of anhydrous sodium carbonate, are placed in a three-necked flask with a stirrer, reflux condenser and dropping funnell; then, a solution of 50 mmoles of chloride of 1-phenyl-cyclopentancarboxylic acid, prepared by reaction of thionyl chloride on the corresponding acid (J. Org. Chem. *4*, 619 (1959)) in 50 ml of benzene, is added in small portions and under stirring. When the addition is complete, the reaction mixture is left under stirring for one hour and is then refluxed for three hours. After hot filtration, benzene is removed under reduced pressure. The residue is re-crystallized from ethanol; m.p. 122—123°C (ethanol).

Analytical and spectroscopical data agree with the teoretical data.

### Example 2

2-[(1-Phenyl)cyclopentyl-carbonyl]-aminopyridine

The reaction is carried out according to the example 1, but using 2-aminopyridine instead of 3-aminopyridine, and substituting sodium carbonate with triethylamine. The product obtained — whose structure is confirmed by analytical and spectroscopical data — melts at 86—87°C (methanol).

Similar results are obtained using, instead of acid chloride, the corresponding mixed anhydride, obtained by 1-phenyl-cyclopentancarboxylic acid and ethyl chlorocarbonate in the presence of a tertiary base.

Starting from such an anhydride, or by 1-phenyl-1-chlorocarbonylcyclopentane, and from the suitable aminopyridine, the following compounds according to the invention are moreover obtained:

— 2-[(1-phenyl)cyclopentyl-carbonyl]-amino-3-methyl-pyridine;
— 2-[(1-phenyl)cyclopentyl-carbonyl]-amino-4-methylpyridine;
— 2-[(1-phenyl)cyclopentyl-carbonyl]-amino-6-methyl-pyridine;

— 2-[(1-phenyl)cyclopentyl-carbonyl]-amino-4,6-dimethyl-pyridine;
— 2-[(1-phenyl)cyclopentyl-carbonyl]-amino-5-chloro-pyridine.

The above compounds, whose characteristics are reported in the following table, show IR spectra having νNH at 3300—3400 $cm^{-1}$ and νCO at 1650—1700 $cm^{-1}$ and NMR spectra having multiplet band at 1.4—1.90 p.p.m. (cycloalkyl hydrogens) and a multiplet band at 6.60—8.20 p.p.m. (benzene and pyridine hydrogens).

TABLE 1

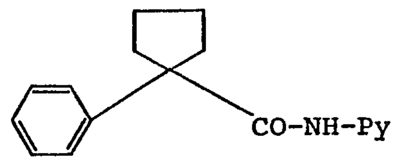

(I)

| Py | M.p. | Yield | Molecular formula | Elementary analysis calc./found | | |
|---|---|---|---|---|---|---|
| | | | | C | H | N |
| 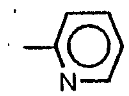 | 86–87°C (methanol) | 80 | $C_{17}H_{18}N_2O$ | 76.66<br>76.64 | 6.81<br>6.79 | 10.52<br>10.48 |
| 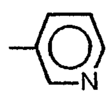 | 122–123°C (ethanol) | 75 | $C_{17}H_{18}N_2O$ | 76.66<br>76.69 | 6.81<br>6.74 | 10.52<br>10.54 |
| 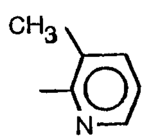 | 106–108°C (ethylacetate) | 80 | $C_{18}H_{20}N_2O$ | 77.11<br>77.06 | 7.19<br>7.17 | 9.99<br>9.94 |

TABLE I (Continued)

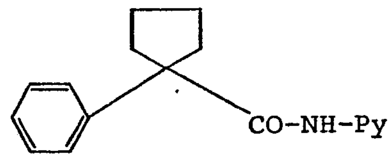

(I)

| Py | M.p. | Yield | Molecular formula | Elementary analysis calc./found C | H | N |
|---|---|---|---|---|---|---|
| 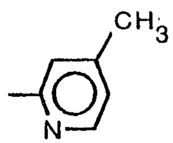 | 99–100°C (ethanol) | 83 | $C_{18}H_{20}N_2O$ | 77.11<br>77.10 | 7.19<br>7.17 | 9.99<br>10.11 |
| 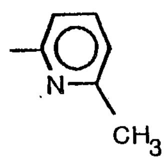 | 103–104°C (ethanol) | 78 | $C_{18}H_{20}N_2O$ | 77.11<br>77.18 | 7.19<br>7.10 | 9.99<br>9.95 |
| 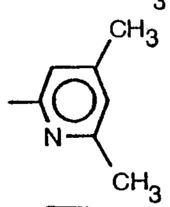 | 80–81°C (methanol) | 85 | $C_{19}H_{22}N_2O$ | 77.52<br>77.48 | 7.53<br>7.54 | 9.52<br>9.55 |
| 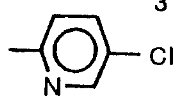 | 99–101°C (ethanol) | 70 | $C_{17}H_{17}ClN_2O$ | 67.88<br>67.90 | 5.65<br>5.69 | 9.32<br>9.40 |

The compounds according to the invention are endowed with limited toxicity together with antiinflammatory, analgesic and antitussive properties.

As an example, pharmacological data for 2-[(1-phenyl)cyclopentyl-carbonyl]-amino-3-methylpyridine which will be also defined, for the sake of brevity, with the abbreviation MR 549, are reported.

*Acute toxicity*

The toxicity after single administrations of MR 549 has been studied in mice and rats by the oral and intraperitoneal routes.

The $LD_{50}$ values have been calculated according to the method of Litchfield and Wilcoxon (J. Pharm. Exp. Therap., 1949, *96*, 99).

From the results obtained, outlined in Table 2, it turned out that MR 549 has a very low acute toxicity, in the used experimental conditions.

TABLE 2

Acute toxicity of MR 549

| Species | Route | $LD_{50}$ mg/kg |
|---|---|---|
| mouse | os<br>i.p. | 550<br>300 |
| rat | os<br>i.p. | 1100<br>600 |

Pharmacological Activity

*Antitussive activity*

The antitussive activity of MR 549 has been studied in the rabbit according to the method of Boura and coll. (Prog. Brit. Pharmac. Soc., apr. 1970) by inducing cough with an aerosol administration of citric acid. MR 549 and an experimented antitussive agent, codeine, as a reference compound, have been administered by intraperitoneal route at equiponderant doses immediately before treating animals with citric acid aerosol. In the 10 minutes following the end of treatment, cough strokes of each animal have been counted, computing the average for each group and the percent inhibition in comparison with a control group.

TABLE 3

Antitussive activity of MR 549

| Treatment | Doses mg/kg i.p. | No. Animals | % Inhibition of Cough Strokes in Comparison with Basal values | Statistical Significance (basal minus final values) |
|---|---|---|---|---|
| Controls | – | 12 | 13.7 | N.S. |
| Codeine | 50 | 12 | 79.5 | P < 0,01 |
| MR 549 | 50 | 12 | 63.5 | P < 0,01 |

From the obtained results reported in Table 3 it turns out that MR 549 has a marked antitussive activity, statistically significant in comparison with control groups and with a very high percentage, comparable to a standard of proved efficacy such as codeine.

*Analgesic activity*

The analgesic properties of MR 549 have been studied with the test of whrithing by phenylquinone in mice according to the method of Siegmund et al. (Proc. Soc. Exp. Biol. Med. 1957, *95*, 729): MR 549 has been administered by the oral route 30 minutes before of the phenylquinone injection at the dose of 50 mg/kg per os; acetylsalicylic acid, drug with proved analgesic activity, has been used as reference drug at the dose of 100 mg/kg per os. From the results obtained and shown in the Table 4 a potent analgesic activity of MR 549 is noticed, comparable to that of acetylsalicylic acid, in the used experimental conditions.

TABLE 4

Analgesic activity of MR 549 — Whrithing by phenylquinone

| Treatment | Doses mg/kg os | Weight g $\bar{X}$ ± e.s. | No. No. Contorsions $\bar{X}$ ± e.s. | % Inhibition in comparison with Controls | No. Whrithing Animals/Total No. |
|---|---|---|---|---|---|
| Controls | — | 26.6 ± 0.58 | 19.1 ± 3.24 | — | 20/20 |
| Acetyl-Salicylic Acid | 100 | 25.8 ± 0.59 | 3.9 ± 1.93 | 79.6 | 12/20 |
| MR 549 | 50 | 25.3 ± 0.79 | 6.9 ± 2.24 | 63.9 | 14/20 |

*Antiinflammatory activity*

The antiinflammatory activity of MR 549 has been assessed by the carrageenin edema test in the rat according to the method of Winter et al. (Proc. Soc. Exp. Biol. Med., 1962, III, 544).

The product under exam and indomethacin at equiponderant doses have been administered by the oral route 30 minutes before of the subplantar carrageening injection; pletismographic measures of the edemas have been performed 3 hours after they had been induced. The results obtained are reported in Table 5.

From the analysis of the obtained data it is possible to notice that MR 549 is endowed with a good antiinflammatory activity, slightly lower than that of indomethacin in the used experimental conditions.

TABLE 5

Antiinflammatory activity — Edema by carrageenin

| Drug | Doses mg/kg | No. Animals | Edema Volume | | % Inhibition |
|---|---|---|---|---|---|
| | | | Basal value | Final value | |
| Controls | — | 10 | 23.4 | 38.6 | — |
| Indo-methacine | 50 | 10 | 22.5 | 29.6 | 53.2 |
| MR 549 | 50 | 10 | 22.6 | 31.8 | 38.1 |

*Gastric tolerability*

The effect on gastric mucosa of MR 549 and of phenylbutazone as a reference drug has been assessed with the method described by Pisanti and Volterra (Il Farmaco Ed. pr. Vol. 25 (2), 105).

Male rats of S—D strain, weighing 180—200 g and fasting for 48 hours with free access to water, have been used. The product under exam have been administered by intraperitoneal route: twice (after 24 and 31 hours from the beginning of the fasting period) at the single dose of 100 mg/kg; or three times by oral route (9.24 and 31 hours after the beginning of the fasting period) at the single dose of 150 mg/kg. The solvent alone (1% carboxymethylcellulose) has been administered to a control group of animals. The results obtained, reported in the Table 6, show that MR 549 is endowed, both by parenteral and oral route, with a gastrolesive action clearly lower than phenylbutazone, according to the used method.

TABLE 6

| Treatment | Dose mg/kg | No. Animals | No. Animals with Ulcers | % Animals with Ulcers |
|---|---|---|---|---|
| Controls (1% carboxy-methyl-cellulose) | — | 12 | 0 | — |
| Phenyl-butazone | 100 mg × 2 i.p. | 12 | 10* | 83.30 |
| Phenyl-butazone | 150 mg × 3 os | 12 | 11** | 91.67 |
| MR 549 | 100 mg × 2 i.p. | 12 | 3 | 25.00 |
| MR 549 | 150 mg × 3 os | 12 | 5 | 41.67 |

*an animal died during treatment.
**two animals died during treatment.

*Motor activity*

The effect of MR 549 on the motor activity has been studied in the Swiss mouse, by placing two groups of animals in a box connected with a recorder detecting spontaneous motility. On the basis of movements/minute recorded, MR 549 at the dose of 50 mg/kg i.p. has been administered to one group and the activity has been recorded during the 2 hours following treatment; the second group, treated with the solvent alone, has been used as control group. The results obtained are reported in Table 7. From analysis of these data it turns out that MR 549 does not change the motor activity in the two hours following treatment.

TABLE 7

| Treatment | Dose | No. recorded movements/min. | | | | |
|---|---|---|---|---|---|---|
| | | Time: 0 | 15' | 30' | 60' | 90' |
| Controls | — | 53 | 48 | 53 | 40 | 44 |
| MR 549 | 50 mg/kg i.p. | 45 | 42 | 52 | 39 | 52 |

The compounds (I) according to the present invention are therefore agents useful for the treatment of cough and of inflammatory conditions, in the form of pharmaceutical compositions such as:

— capsules, pills or tablets containing 100—500 mg of compounds (I);

— syrup in such a concentration to deliver 300—1500 mg of compounds (I) a day, when administered b.i.d., t.i.d. or more times;

— suppositories containing 200—1000 mg of compounds (I).

**Claims for the Contracting States: BE CH DE FR GB IT LI LU NL SE**

1. N-Pyridyl-amides of 1-phenyl-cyclopentancarboxylic acid having formula (I)

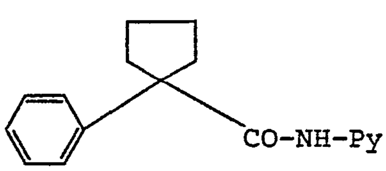

(I)

wherein Py is the pyridine ring, optionally substituted with one or more methyl groups or chlorine atoms.

2. As a compound according to claim 1, 2-[(1-phenyl)cyclopentyl-carbonyl]-aminopyridine.
3. As a compound according to claim 1, 3-[(1-phenyl)cyclopentyl-carbonyl]-aminopyridine.
4. As a compound according to claim 1, 2-[(1-phenyl)cyclopentyl-carbonyl]-amino-4-methyl-pyridine.
5. As a compound according to claim 1, 2-[(1-phenyl)cyclopentyl]-amino-6-methyl-pyridine.
6. As a compound according to claim 1, 2-[(1-phenyl)cyclopentyl-carbonyl]-amino-3-methyl-pyridine.
7. As a compound according to claim 1, 2-[(1-phenyl)cyclopentyl-carbonyl]-amino-4,6-dimethyl pyridine.
8. As a compound according to claim 1, 2-[(1-phenyl)cyclopentyl-carbonyl]-amino-5-chloropyridine.
9. Process for the preparation of compounds according to claims 1—8, characterized in that activated derivatives of 1-phenyl-cyclopentan-carboxylic acid, having general formula (II), are reacted with aminopyridines (III), according to the schemes.

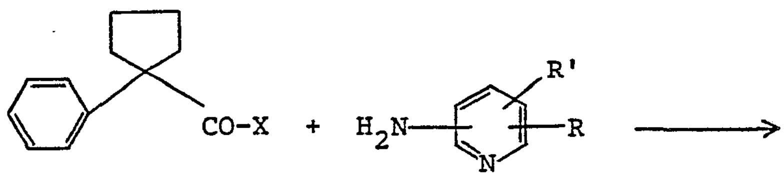

(I)

(II) (III)

wherein X is an halogen atom, or an alcoxy group, or a residue transforming (II) in a symmetrical or mixed anhydride; R and R′, which can be the same or different, are hydrogen atoms, methyl groups or chlorine atoms.

10. Pharmaceutical compositions endowed with antitussive and antiinflammatory activity, characterized by containing as an active principle a compound according to claims 1—8.

**Claims for the Contracting State: AT**

1. Process for the preparation of compounds having formula I

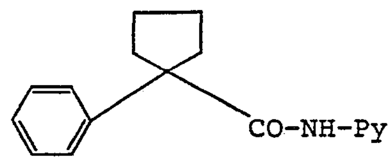

(I)

wherein Py is the pyridine ring, optionally substituted with one or more methyl groups or chlorine atoms characterized in that activated derivatives of 1-phenyl-cyclopentan-carboxylic acid, having general formula (II), are reacted with aminopyridines (III), according to the schemes

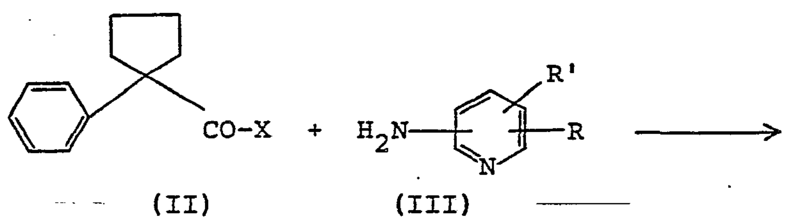

(I)

(II) (III)

wherein X is an halogen atom, or an alcoxy group, or a residue transforming (II) in a symmetrical or mixed anhydride; R and R′, which can be the same or different, are hydrogen atoms, methyl groups or chlorine atoms.

2. Process according to claim 1, characterized in that the reaction is carried out in inert solvents such as ethylether, dioxane, chloroform, benzene or toluene.

3. Process according to claim 1, characterized in that, when X is halogen, an hydrohalic acid acceptor or an excess of aminopyridine (III) are used.

**Patentansprüche für die Vertragsstaaten: BE CH DE FR GB IT LI LU NL SE**

1. N-Pyridylamide von 1-Phenylcyclopentan-carbonsäure der allgemeinen Formel I

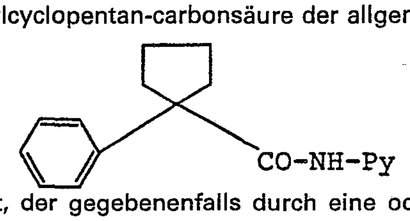

(I)

worin Py den Pyridinring bedeutet, der gegebenenfalls durch eine oder mehrere Methylgruppen oder Chloratome substituiert ist.

2. 1,2-[(1-Phenyl)cyclopentyl-carbonyl]-aminopyridin.
3. 1,3-[(1-Phenyl)cyclopentyl-carbonyl]-aminopyridin.
4. 1,2-[(1-Phenyl)cyclopentyl-carbonyl]-amino-4-methyl-pyridin.
5. 1,2-[(1-Phenyl)cyclopentyl-carbonyl]-amino-6-methyl-pyridin.
6. 1,2-[(1-Phenyl)cyclopentyl-carbonyl]-amino-3-methyl-pyridin.
7. 1,2-[(1-Phenyl)cyclopentyl-carbonyl]-amino-4,6-dimethyl-pyridin.
8. 1,2-[(1-Phenyl)cyclopentyl-carbonyl]-amino-5-chlor-pyridin.
9. Verfahren zur Herstellung der Verbindungen nach den Ansprüchen 1 bis 8, dadurch gekennzeichnet, daß aktivierte Derivate von 1-Phenyl-cyclopentan-carbonsäure der allgemeinen Formel II mit Aminopyridinen der allgemeinen Formel III umgesetzt werden nach dem Schema

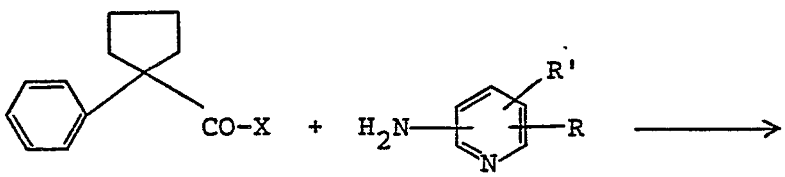

(I)

(II) (III)

worin X ein Halogenatom oder einen Alkoxyrest bedeutet oder einen Rest, der II in ein symmetrisches oder gemischtes Anhydrid überführt, und R und R', die gleich oder unterschiedlich sein können, Wasserstoffatome, Methylgruppen oder Chloratome darstellen.

10. Pharmazeutische Zusammensetzungen mit hustenreizmildernder und entzündungshemmender Wirkung, gekennzeichnet durch einen Gehalt an einer Zusammensetzung nach einem der Ansprüche 1 bis 8 als aktiven Wirkstoff.

**Patentansprüche für den Vertragsstaat: AT**

1. Verfahren zur Herstellung von Verbindungen der allgemeinen Formel I

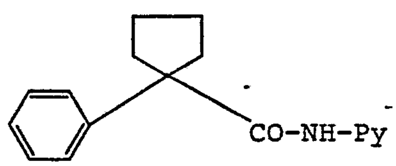

(I)

worin Py den Pyridinring bedeutet, der gegebenenfalls durch eine oder mehrere Methylgruppen oder Chloratome substituiert ist, dadurch gekennzeichnet, daß aktivierte Derivate von 1-Phenyl-cyclopentan-carbonsäure der allgemeinen Formel II mit Aminopyridinen der allgemeinen Formel III umgesetzt werden nach dem folgenden Schema

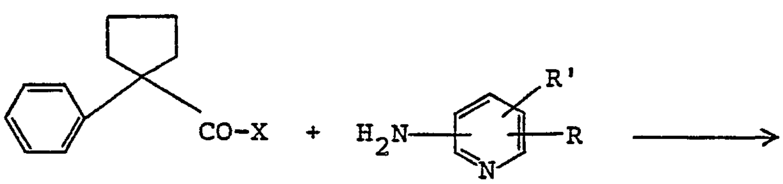

(I)

(II) (III)

worin X ein Halogenatom oder eine Alkoxygruppe bedeutet oder einen Rest, der II in ein symmetrisches oder gemischtes Anhydrid überführt, und R und R', die gleich oder unterschiedlich sein können, Wasserstoffatome, Methylgruppen oder Chloratome bedeuten.

2. Verfahren nach Anspruch 1, dadurch gekennzeichnet, daß die Reaktion in inerten Lösungsmitteln durchgeführt wird, wie in Ethylether, Dioxan, Chloroform, Benzol oder Toluol.

3. Verfahren nach Anspruch 1, dadurch gekennzeichnet, daß für den Fall, daß X ein Halogenatom ist, ein Halogenwasserstoffsäureakzeptor oder ein Überschuß von Aminopyridin (III) verwendet wird.

**Revendications pour les Etats contractants: BE CH DE FR GB IT LI LU NL SE**

1. N-Pyridyl-amides de l'acide 1-phényl-cyclopentan-carboxylique de formule (I)

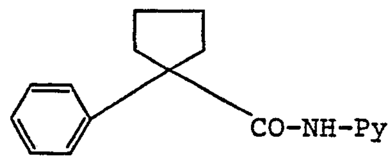

(I)

dans laquelle Py est le cycle pyridine, le cas échéant substitué par un ou plusieurs groupes ou des atomes de chlore.

2. En tant que composé selon la revendication 1, la 2-[(1-phényl)cyclopentyl-carbonyl]-aminopyridine.

3. En tant que composé selon la revendication 1, la 3-[(1-phényl)cyclopentyl-carbonyl]-aminopyridine.

4. En tant que composé selon la revendication 1, la 2-[(1-phényl)cyclopentyl-carbonyl]-amino-4-méthylpyridine.

5. En tant que composé selon la revendication 1, la 2-[(1-phényl)cyclopentyl-carbonyl]-amino-6-méthylpyridine.

6. En tant que composé selon la revendication 1, la 2-[(1-phényl)cyclopentyl-carbonyl]-amino-3-méthylpyridine.

7. En tant que composé selon la revendication 1, la 2-[(1-phényl)cyclopentyl-carbonyl]-amino-4,6-diméthylpyridine.

8. En tant que composé selon la revendication 1, la 2-[(1-phényl)cyclopentyl-carbonyl]-amino-5-chloropyridine.

9. Procédé pour la préparation de composés selon les revendications 1 à 8 caractérisées en ce qu'on fait réagir les dérivés activés de l'acide, 1-phénylcyclopentan-carboxylique de formule générale (II) avec des aminopyridines (III), selon les schémas

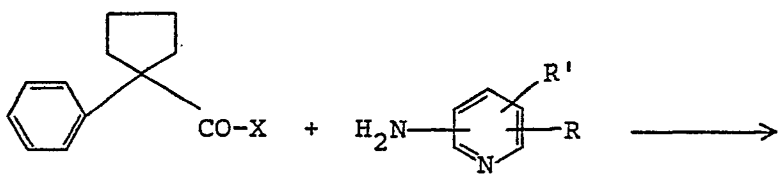

(I)

(II) (III)

dans lesquels X est un atome d'halogène ou un groupe alcoxy, ou un résidu transformant (II) en une anhydride homogène symétrique ou mixte; R et R' qui peuvent être identiques ou différents, sont des atomes d'hydrogène, des groupes méthyle ou des atomes de chlore.

10. Compositions pharmaceutiques dotées d'une activité antitussive et antiinflammatoire, caractérisées en ce qu'elles contiennent comme principe actif un composé selon les revendications 1—8.

**Revendications pour l'Etat contractant: AT**

1. Procédé pour la préparation de composés de formule (I)

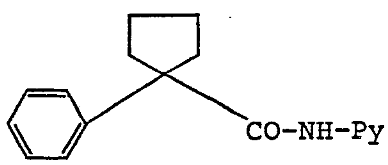

(I)

dans laquelle Py est le cycle pyridine, le cas échéant substitué par un ou plusieurs groupes méthyle ou des atomes de chlore, caractérisées en ce qu'on fait réagire les dérivés activés de l'acide, 1-phényl-cyclopentan-carboxylique de formule générale (II) avec des aminopyridines (III), selon les schémas

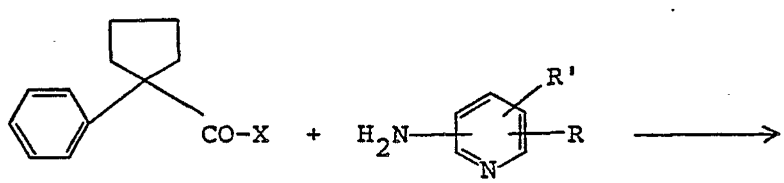

(I)

(II) (III)

dans lesquels X est un atome d'halogène ou un groupe alcoxy, ou un résidu transformant (II) en une

anhydride homogène symétrique ou mixte; R et R' qui peuvent être identiques ou différents, sont des atomes d'hydrogène, des groupes méthyle ou des atomes de chlore.

2. Procédé selon la revendication 1, caractérisées en ce que la réaction est menée dans un solvant inerte tels que éther éthylique, dioxanne, chloroforme, benzène ou toluène.

3. Procédé selon la revendication 1, caractérisées en ce qu'on utilise un accepteur acide hydroalcoolique ou un excès de pyramidon (III) quand X est un atome d'halogène.